# FASCICULE DE BREVET EUROPEEN

(11) **EP 0 779 883 B1**
(45) Date de publication et mention de la délivrance du brevet: **16.08.2000**
(21) Numéro de dépôt: 96904139.1
(22) Date de dépôt: 14.02.1996
(51) Int. Cl.: C07C 45/67, C07C 47/575, C07C 47/58, C07C 65/21, C07C 51/15, C07C 51/367, C07C 51/373

(54) **PROCEDE DE PREPARATION D'UN 4-HYDROXYBENZALDEHYDE SUBSTITUE**
VERFAHREN ZUR HERSTELLUNG VON EINEM SUBSTITUIERTEN 4-HYDROXYBENZALDEHYD
METHOD FOR PREPARING A SUBSTITUTED 4-HYDROXYBENZALDEHYDE

(30) Priorité: 20.02.1995 FR 9501926
(43) Date de publication de la demande: 25.06.1997
(73) Titulaire: RHODIA CHIMIE, 92408 Courbevoie Cédex (FR)
(72) Inventeur: METIVIER, Pascal, F-69110 Sainte-Foy-lès-Lyon (FR); JOUVE, Isabelle, F-69740 Genas (FR); MALIVERNEY, Christian, F-69007 Lyon (FR)
(74) Mandataire: Dutruc-Rosset, Marie-Claude
(86) Numéro de dépôt international: FR9600241
(87) Numéro de publication internationale: WO9626175

(56) Documents cités:
- DE-C- 51 381
- DE-C- 71 162
- DE-C- 72 600
- ARCH. MICROBIOL. (AMICCW,03028933);79; VOL.121 (1); PP.23-8, KYOTO UNIV.;WOOD RES. INST.; UJI; JAPAN, XP002003065 OHTA M ET AL: "Microbial degradation of dehydrodiconiferyl alcohol, a lignin substructure model"

## Description

La présente invention a pour objet un procédé de préparation d'un 4-hydroxybenzaldéhyde porteur d'au moins un substituant en position ortho du groupe OH.

Elle concerne plus particulièrement la préparation du 3-méthoxy-4-hydroxybenzaldéhyde et du 3-éthoxy-4-hydroxybenzaldéhyde dénommés respectivement "vanilline" et "éthylvanilline".

La vanilline est obtenue principalement à partir de sources naturelles telles que la lignine mais une partie est préparée par voie chimique.

De nombreuses méthodes de préparation sont décrites dans la littérature [KIRK-OTHMER - Encyclopedia of Chemical Technology 23, p 1710, 3^{ème} édition] et plusieurs d'entre elles partent du gaïacol ou 2-méthoxyphénol.

Ainsi, on peut mentionner la préparation de la vanilline par réaction du gaïacol et de l'acide glyoxylique, oxydation à l'air du condensat, puis libération de la vanilline du milieu réactionnel par acidification. L'inconvénient dont souffre ce procédé est de faire appel à l'acide glyoxylique qui est un réactif coûteux.

Une autre voie d'accès à la vanilline selon la réaction de Reimer-Tiemann consiste à faire réagir le gaïacol et le chloroforme en présence d'hydroxyde de potassium. La formation de résine est un désavantage de cette méthode de préparation.

Selon la réaction de Gatterman, la vanilline est synthétisée par action de l'acide cyanhydrique sur le gaïacol, en présence d'acide chlorhydrique. Outre la mise en oeuvre d'un réactif de manipulation délicate, ce procédé présente l'inconvénient de ne pas être sélectif car la vanilline est accompagnée d'isovanilline et d'o-vanilline.

Par ailleurs, il est décrit dans Arch. Microbiol. 121 (1979), l'obtention dans le métabolisme de l'alcool-5-carboxyvanillique à partir d'alcool 5-acétylvanillique.

Le brevet allemand DE-C-72600 mentionne l'obtention de vanilline à partir de 3-carboxy-4-hydroxy-5-méthoxybenzaldéhyde.

Une difficulté majeure présente dans la synthèse de la vanilline est de fixer un groupe formyle sur le gaïacol, sélectivement en position para du groupe hydroxyle.

Un autre problème à résoudre est de fournir un procédé compétitif d'un point de vue industrielle.

La présente invention propose un nouveau procédé permettant d'obvier aux inconvénients précités tout en satisfaisant aux exigences mentionnées ci-dessus.

Il a maintenant été trouvé et c'est ce qui constitue l'objet de la présente invention, un procédé de préparation d'un 4-hydroxybenzaldéhyde substitué, au moins en position 3 par un groupe alkoxy, caractérisé par le fait qu'il consiste à soumettre un composé phénolique substitué au moins en position 2 par un groupe alkoxy et dont les positions 4 et 6 sont libres, à une première étape de carboxylation en position 6 ; puis à une étape d'hydroxyméthylation en position 4, suivie d'une étape d'oxydation du groupe hydroxyméthyle en groupe formyle ; enfin à une dernière étape de décarboxylation.

Le procédé de l'invention est fondé sur la préparation des acides 2-hydroxybenzoïques hydroxyméthylés en position 5 et substitués au moins en position 3 par un groupe alkoxy, qui servent de produits intermédiaires dans la synthèse de 4-hydroxybenzaldéhydes substitués, au moins en position 3 par d'un groupe alkoxy.

Un autre objet de l'invention est le procédé d'oxydation des acides 2-hydroxybenzoïques hydroxyméthylés en position 5 et substitués, au moins en position 3 par un groupe alkoxy, en acides 2-hydroxybenzoïques formylés correspondants.

Le procédé de l'invention convient tout-à-fait bien à la préparation de la vanilline. En effet, il permet d'effectuer la formylation du gaïacol sélectivement en position para en effectuant successivement une carboxylation du gaïacol en position 6, une hydroxyméthylation suivie d'une oxydation conduisant au groupe formyle en position 4 et enfin une élimination du groupe carboxylique situé en position 6.

Ce procédé n'est pas seulement sélectif mais aussi très compétitif d'un point de vue industriel car il fait appel à des réactifs peu onéreux.

Bien que le procédé de l'invention soit parfaitement bien adapté à la mise en oeuvre du gaïacol et du guétol, il convient également pour d'autres composés phénoliques substitués.

Par "composé phénolique substitué", on entend tout composé aromatique, dont le noyau aromatique est porteur d'un groupe hydroxyle, d'un groupe alkoxy en position 2, d'autres substituants éventuels et dont les positions 4 et 6 sont libres.

Dans l'exposé qui suit de la présente invention, on entend par "aromatique", la notion classique d'aromaticité telle que définie dans la littérature, notamment par Jerry MARCH, Advanced Organic Chemistry, 4^{ème} édition, John Wiley and Sons, 1992, pp. 40 et suivantes.

Parmi les composés phénoliques substitués, l'invention s'applique tout particulièrement à ceux qui répondent à la formule générale (I): 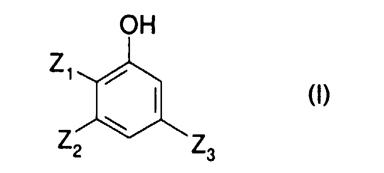 dans ladite formule (I):
- Z₁ représente :
   . un radical alkoxy linéaire ou ramifié ayant de 1 à 12 atomes de carbone, de préférence de 1 à 4 atomes de carbone tel que les radicaux méthoxy, éthoxy, propoxy, isopropoxy, butoxy, isobutoxy, sec-butoxy, tert-butoxy,
- Z₂ et Z₃, identiques ou différents, représentent un atome d'hydrogène ou l'un des groupes suivants :
   . un radical alkyle, linéaire ou ramifié, ayant de 1 à 12 atomes de carbone, de préférence de 1 à 4 atomes de carbone, tel que méthyle, éthyle, propyle, isopropyle, butyle, isobutyle, sec-butyle, tert-butyle,
   . un radical alcényle, linéaire ou ramifié, ayant de 2 à 12 atomes de carbone, de préférence de 2 à 4 atomes de carbone, tel que vinyle, allyle,
   . un radical alkoxy linéaire ou ramifié ayant de 1 à 12 atomes de carbone, de préférence de 1 à 4 atomes de carbone tel que les radicaux méthoxy, éthoxy, propoxy, isopropoxy, butoxy, isobutoxy, sec-butoxy, tert-butoxy,
   . un radical phényle,
   . un atome d'halogène, de préférence un atome de fluor, chlore ou brome.

La présente invention n'exclut pas la présence sur le cycle aromatique de substituants d'une nature différente dans la mesure où ils n'interfèrent pas avec les réactions du procédé de l'invention.

La présente invention s'applique préférentiellement aux composés de formule (I) dans laquelle Z₁ représente un radical alkoxy, linéaire ou ramifié, ayant de 1 à 6 atomes de carbone, de préférence, 1 à 4 atomes de carbone ; Z₂ et Z₃ représentant un atome d'hydrogène.

Comme exemples préférés de substrats mis en oeuvre dans le procédé de l'invention, on peut citer, entre autres, le gaïacol et le guétol.

Conformément au procédé de l'invention, on part d'un composé phénolique substitué qui est préférentiellement un composé répondant à la formule (I).

On donne ci-après, le schéma réactionnel du procédé de l'invention pour faciliter la compréhension de l'exposé de l'invention, sans pour autant lier la portée de l'invention, à celui-ci.

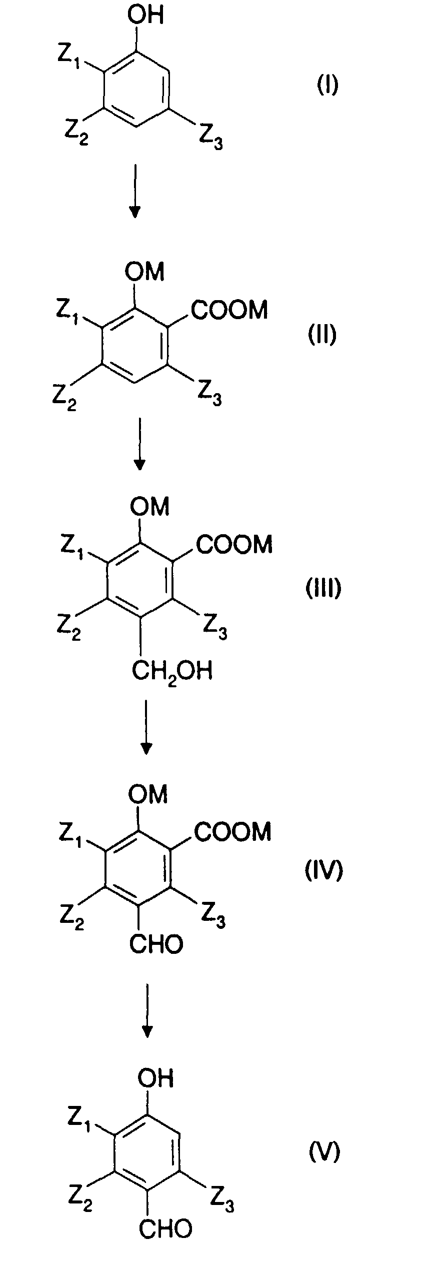

Dans l'exposé qui suit de la présente invention, on va se référer aux formules données ci-après, sans pour autant limiter l'invention aux substrats définis par les formules données.

Conformément au procédé de l'invention, on effectue dans une première étape, la carboxylation d'un composé phénolique substitué de formule (I), par réaction dudit composé phénolique substitué, sous forme salifiée avec du dioxyde de carbone.

Les composés phénoliques substitués interviennent donc dans le procédé de l'invention sous forme salifiée. Il s'agit préférentiellement des sels des éléments métalliques du groupe (la) de la classification périodique.

Pour la définition des éléments, on se réfère ci-après à la Classification périodique des éléments publiée dans le Bulletin de la Société Chimique de France, n°1 (1966).

D'un point de vue pratique et économique, on fait appel aux sels de sodium ou de potassium.

Conformément au procédé de l'invention, on fait réagir le composé phénolique substitué sous forme salifiée et le dioxyde de carbone.

On peut faire appel à une forme salifiée d'un composé phénolique substitué préparée extemporanément mais il est également possible de la préparer in situ en faisant réagir le composé phénolique substitué avec une base.

Intervient donc, dans le procédé de l'invention, une base qui peut être minérale ou organique.

On choisit préférentiellement une base forte c'est-à-dire une base ayant un pKb supérieur à 12 : le pKb étant défini comme le cologarithme de la constante de dissociation de la base mesurée, en milieu aqueux, à 25°C.

Conviennent particulièrement bien à la mise en oeuvre du procédé de l'invention, les bases minérales telles que les sels de métaux alcalins, de préférence, un hydroxyde de métal alcalin qui peut être l'hydroxyde de sodium ou de potassium ; un carbonate de métal alcalin, de préférence, de potassium.

Il est également possible de faire appel à un hydroxyde d'ammonium quaternaire.

Comme exemples d'hydroxyde d'ammonium quaternaire, on met en oeuvre préférentiellement, les hydroxydes de tétralkylammonium ou de trialkylbenzylammonium dont les radicaux alkyle identiques ou différents représentent une chaîne alkyle linéaire ou ramifiée ayant de 1 à 12 atomes de carbone, de préférence de 1 à 6 atomes de carbone.

On choisit préférentiellement l'hydroxyde de tétraméthylammonium, l'hydroxyde de tétraéthylammonium ou l'hydroxyde de tétrabutylammonium.

Il est également possible selon l'invention d'avoir recours à des hydroxydes de trialkylbenzylammonium et notamment à l'hydroxyde de triméthylbenzylammonium.

Pour des considérations économiques, on choisit parmi toutes les bases, préférentiellement l'hydroxyde de sodium ou le carbonate de potassium.

La concentration de la solution basique de départ n'est pas critique. La solution d'hydroxyde de métal alcalin mise en oeuvre a une concentration généralement comprise entre 10 et 50 % en poids.

La quantité de base introduite dans le milieu réactionnel tient compte de la quantité nécessaire pour salifier la fonction hydroxyle du composé phénolique substitué.

Si ledit composé présente des fonctions salifiables autres que le groupe hydroxyle, on introduit donc la quantité de base nécessaire pour salifier toutes les fonctions salifiables.

Généralement, la quantité de base exprimée par rapport au composé phénolique substitué varie entre 90 et 120 % de la quantité stoechiométrique.

On prépare le composé phénolique substitué sous forme salifiée en le faisant réagir avec la base à une température avantageusement comprise entre 25°C et 100°C.

Avant d'introduire le dioxyde de carbone, on élimine l'eau formée au cours de la réaction de salification, par distillation sous pression atmosphérique ou sous une pression réduite comprise entre 1 mm de mercure et la pression atmosphérique ou par séchage. Lorsqu'il n'y a plus d'eau dans le milieu, on introduit le dioxyde de carbone.

Une autre variante du procédé de l'invention consiste à mettre en oeuvre en plus, un carbonate de métal alcalin anhydre, de préférence, le carbonate de potassium anhydre ce qui permet d'éviter l'étape de l'élimination de l'eau (par exemple de 5 à 100 % en moles de composé phénolique substitué).

La quantité de dioxyde de carbone à mettre en oeuvre exprimée par le rapport molaire entre le dioxyde de carbone et le composé phénolique substitué varie entre 1 et 100, et plus préférentiellement entre 1 et 2.

Le procédé de l'invention est avantageusement conduit à une température comprise entre 150°C et 250°C, de préférence, entre 160°C et 200°C.

Il est généralement mis en oeuvre sous pression atmosphérique, en faisant buller le dioxyde de carbone dans le milieu réactionnel maintenu sous agitation.

Il est également possible de conduire la réaction sous pression de dioxyde de carbone variant entre la pression atmosphérique et environ 100 bar. On préfère une pression entre 1 et 20.

Un mode préféré de réalisation pratique de l'invention consiste à mettre en oeuvre le composé phénolique substitué, la base, à éliminer l'eau si nécessaire par distillation puis à introduire le dioxyde de carbone. Il s'agit d'une réaction solide/gaz.

En fin de réaction, on récupère alors, le composé phénolique substitué porteur d'un groupe carboxylate en position 6 et on le met en solution dans l'eau à une concentration variant entre 5 et 50 % en poids.

On amène la solution à un pH compris entre environ 6 et 10, de préférence, voisin de 7, par addition d'un acide.

On peut utiliser n'importe quel acide mais pour des considérations économiques, on préfère faire appel aux acides minéraux classiques, de préférence, l'acide chlorhydrique ou l'acide sulfurique. La concentration de l'acide mis en jeu n'est pas critique. Elle correspond de préférence à la concentration de la forme commerciale, par exemple, 37 % en poids pour l'acide chlorhydrique, 92 ou 96 % pour l'acide sulfurique.

On obtient alors un milieu bi-phasique constitué d'une phase organique comprenant le composé phénolique substitué qui n'a pas réagi et d'autre part une phase aqueuse comprenant le produit souhaité à savoir le sel de l'acide 2-hydroxybenzoïque au moins substitué, en position 3 par un groupe alkoxy qui est représenté par la formule (Il) dans laquelle Z₁, Z₂ et Z₃ ont les significations données précédemment et M représente un atome d'hydrogène et/ou un cation métallique du groupe (la).

Il est dénommé par la suite, de manière simplifiée par "sel d'acide hydroxybenzoïque".

On sépare les deux phases et l'on recueille la phase aqueuse.

Il est à noter que l'on ne sortira pas du cadre de la présente invention de partir du sel d'acide hydroxybenzoïque de formule (II) préparé extemporanément.

Dans une étape suivante, on effectue une réaction d'hydroxyméthylation en position para du groupe OH par réaction du sel de l'acide précédemment obtenu avec du formaldéhyde, éventuellement en présence d'une base.

On peut faire appel au formaldéhyde ou tout générateur de formaldéhyde tel que, par exemple, le trioxane ou le paraformaldéhyde utilisé sous la forme de polyformaldéhydes linéaires de degré de polymérisation indifférent, ayant de préférence un nombre de motifs (CH₂O) compris entre 8 et 100 motifs,

On met en oeuvre ledit réactif généralement sous forme d'une solution aqueuse ayant une concentration inférieure à 50 % en poids, de préférence, comprise entre 20 et 50 % en poids.

La quantité de formaldéhyde exprimée en moles de formaldéhyde par mole de sel d'acide hydroxybenzoïque peut varier dans de larges limites. Le rapport molaire formaldéhyde/sel d'acide hydroxybenzoïque se situe avantageusement entre 0,5 et 3,0.

Il est possible de conduire la réaction en présence d'une base. Les bases mentionnées précédemment conviennent tout à fait bien.

La quantité de base mise en oeuvre exprimée par le rapport entre le nombre de moles de base et le nombre de moles de sel d'acide hydroxybenzoïque peut varier entre 0 et 2, et de préférence, entre 0 et 1,1.

La base peut être mise en oeuvre indifféremment sous forme solide ou en solution aqueuse.

La température de la réaction peut varier de 50°C à 100°C et, de préférence, de 60°C à 80°C.

Le procédé est conduit, de préférence, sous la pression autogène des réactifs pour éviter les pertes éventuelles du paraformaldéhyde qui peut être gazeux aux températures de mises en oeuvre.

On préfère conduire la réaction sous atmosphère contrôlée de gaz inertes tels que l'azote ou les gaz rares, par exemple l'argon.

La durée de la réaction peut être très variable. Elle se situe, le plus souvent, entre 30 minutes et 24 heures, de préférence entre 4 heures et 8 heures.

D'un point de vue pratique, la réaction est aisément réalisée en chargeant dans l'appareillage le sel d'acide hydroxybenzoïque et le formaldéhyde, éventuellement une base, puis en portant le mélange réactionnel sous agitation à la température désirée, pendant la durée nécessaire à l'achèvement de la réaction.

L'ordre d'introduction des réactifs n'est pas critique et peut donc être différent.

En fin de réaction, on obtient un acide 2-hydroxybenzoïque hydroxyméthylé en position 5 et substitué, au moins en position 3 par un groupe alkoxy répondant préférentiellement à la formule (III) dans laquelle Z₁, Z₂ et Z₃ ont les significations données précédemment et M représente un atome d'hydrogène et/ou un cation métallique du groupe (la).

Selon une variante préférée du procédé de l'invention, on ne sépare pas le composé obtenu mais on le soumet directement à une oxydation.

Un mode préféré d'oxydation de l'invention et qui constitue un autre objet de la présente invention consiste à oxyder l'acide 2-hydroxybenzoïque hydroxyméthylé en position 5 et substitué, au moins en position 3 par un groupe alkoxy, en phase liquide, à l'aide d'oxygène moléculaire ou un gaz en contenant, en opérant en milieu aqueux renfermant un agent alcalin, en présence d'un catalyseur à base de platine ou de palladium éventuellement en présence d'un co-catalyseur à base d'un dérivé du bismuth.

En ce qui concerne les métaux nobles utilisés pour catalyser la réaction, en l'occurence le platine et le palladium, ils peuvent avoir diverses formes comme par exemple : le noir de platine, le noir de palladium, l'oxyde de platine, l'oxyde de palladium ou le métal noble lui-même déposé sur des supports divers tels que le noir de carbone, le carbonate de calcium, les alumines et silices activées ou des matériaux équivalents. Des masses catalytiques à base de noir de carbone conviennent particulièrement.

La quantité de ce catalyseur à mettre en oeuvre, exprimée en poids de platine ou palladium par rapport à celui de l'acide 2-hydroxybenzoïque hydroxyméthylé, peut varier de 0,01 à 4 % et, de préférence, de 0,04 à 2 %.

On peut faire appel à un co-catalyseur et plus particulièrement, on fait appel en général à un dérivé minéral ou organique du bismuth dans lequel l'atome de bismuth se trouve à un degré d'oxydation supérieur à zéro, par exemple égal à 2, 3, 4 ou 5. Le reste associé au bismuth n'est pas critique dès l'instant qu'il satisfait à cette condition. Le co-catalyseur peut être soluble ou insoluble dans le milieu réactionnel.

Des composés illustratifs de co-catalyseurs qui peuvent être utilisés dans le procédé selon la présente invention sont : les oxydes de bismuth ; les hydroxydes de bismuth ; les sels d'hydracides minéraux tels que : chlorure, bromure, iodure, sulfure, séléniure, tellure de bismuth ; les sels d'oxyacides minéraux tels que : sulfite, sulfate, nitrite, nitrate, phosphite, phosphate, pyrophosphate, carbonate, perchlorate, antimoniate, arséniate, sélénite, séléniate de bismuth ; les sels d'oxyacides dérivés de métaux de transition tels que : vanadate, niobate, tantalate, chromate, molybdate, tungstate, permanganate de bismuth.

D'autres composés appropriés sont également des sels d'acides organiques aliphatiques ou aromatiques tels que : acétate, propionate, benzoate, salicylate, oxalate, tartrate, lactate, citrate de bismuth ; des phénates tels que : gallate et pyrogallate de bismuth. Ces sels et phénates peuvent être aussi des sels de bismuthyle.

Comme autres composés minéraux ou organiques, on peut utiliser des combinaisons binaires du bismuth avec des éléments tels que phosphore et arsenic ; des hétéropolyacides contenant du bismuth ainsi que leurs sels ; conviennent également les bismuthines aliphatiques et aromatiques.

A titre d'exemples spécifiques, on peut citer :
- comme oxydes : BiO ; Bi₂O₃ : Bi₂O₄ ; Bi₂O₅.
- comme hydroxydes : Bi(OH)₃,
- comme sels d'hydracides minéraux : le chlorure de bismuth BiCl₃ ; le bromure de bismuth BiBr₃ ; l'iodure de bismuth Bil₃ ; le sulfure de bismuth Bi₂S₃ ; le séléniure de bismuth Bi₂Se₃ : le tellure de bismuth Bi₂Te₃,
- comme sels d'oxyacides minéraux : le sulfite basique de bismuth Bi₂(SO₃)₃, Bi₂O₃, 5H₂O ; le sulfate neutre de bismuth Bi₂(SO₄)₃ ; le sulfate de bismuthyle (BiO)HSO₄ ; le nitrite de bismuthyle (BiO)NO₂, 0,5H₂O ; le nitrate neutre de bismuth Bi(NO₃)₃, 5H₂O ; le nitrate double de bismuth et de magnésium 2Bi(N0₃)₃, 3Mg(NO₃)₂, 24H₂O ; le nitrate de bismuthyle (BiO)NO₃ ; le phosphite de bismuth Bi₂(PO₃H)₃, 3H₂O ; le phosphate neutre de bismuth BiPO₄ ; le pyrophosphate de bismuth Bi₄(P₂O₇)₃ ; le carbonate de bismuthyle (BiO)₂CO₃, O,5H₂O ; le perchlorate neutre de bismuth Bi(ClO₄)₃, 5H₂O ; le perchlorate de bismuthyle (BiO)ClO₄ ; l'antimoniate de bismuth BiSbO₄ ; l'arséniate neutre de bismuth Bi(AsO₄)₃ ; l'arséniate de bismuthyle (BiO)AsO₄, 5H₂O ; le sélénite de bismuth Bi₂(SeO₃)₃.
- comme sels d'oxyacides dérivés de métaux de transtion : le vanadate de bismuth BiVO₄ ; le niobate de bismuth BiNbO : le tantalate de bismuth BiTaO₄ ; le chromate neutre de bismuth Bi₂(CrO₄) ; le dichromate de bismuthyle (BiO)₂)₂Cr₂O₇ ; le chromate acide de bismuthyle H(BiO)CrO₄ ; le chromate double de bismuthyle et de potassium K(BiO)CrO₁₀ ; le molybdate de bismuth Bi₂(MoO₄)₃ ; le tungstate de bismuth Bi₂(WO₄)₃ ; le molybdate double de bismuth et de sodium NaBi(MoO₄)₂ ; le permanganate basique de bismuth Bi₂O₂(OH)MnO₄.
- comme sels d'acides organiques aliphatiques ou aromatiques : l'acétate de bismuth Bi(C₂H₃O₂)₃ ; le propionate de bismuthyle (BiO)C₃H₅O₂ ; le benzoate basique de bismuth C₆H₅CO₂Bi(OH)₂ ; le salicylate de bismuthyle C₆H₄CO₂(BiO)(OH) ; l'oxalate de bismuth (C₂O₄)₃Bi₂ ; le tartrate de bismuth Bi₂(C₄H₄O₆)₃, 6H₂O ; le lactate de bismuth (C₆H₉O₅)OBi, 7H₂O ; le citrate de bismuth C₆H₅O₇Bi.
- comme phénates : le gallate basique de bismuth C₇H₇O₇Bi ; le pyrogallate basique de bismuth C₆H₃(OH)₂(OBi)(OH).

Comme autres composés minéraux ou organiques conviennent également : le phosphure de bismuth BiP ; l'arséniure de bismuth Bi₃As₄ ; le bismuthate de sodium NaBiO₃ ; les acides bismuth-thiocyaniques H₂[Bi(BNS)₅], H₃[Bi(CNS)₆] et leurs sels de sodium et potassium ; la triméthylbismuthine Bi(CH₃)₃, la triphénylbismuthine Bi(C₆H₅)₃.

Les dérivés du bismuth qui sont utilisés de préférence pour conduire le procédé selon l'invention sont : les oxydes de bismuth ; les hydroxydes de bismuth ; les sels de bismuth ou de bismuthyle d'hydracides minéraux ; les sels de bismuth ou de bismuthyle d'oxyacides minéraux ; les sels de bismuth ou de bismuthyle d'acides organiques aliphatiques ou aromatiques ; et les phénates de bismuth ou de bismuthyle.

Un groupe de co-catalyseurs qui conviennent particulièrement bien à la réalisation de l'invention est constitué par : les oxydes de bismuth Bi₂O₃ et Bi₂O₄; l'hydroxyde de bismuth Bi(OH)₃; le sulfate neutre de bismuth Bi₂(SO4)₃ ; le chlorure de bismuth BiCl₃ ; le bromure de bismuth BiBr₃ ; l'iodure de bismuth Bil₃ ; le nitrate neutre de bismuth Bi(NO₃)₃, 5H₂O ; le nitrate de bismuthyle BiO(NO₃) ; le carbonate de bismuthyle (BiO)₂CO₃, O,5H₂O ; l'acétate de bismuth Bi(C₂H₃O₂)₃ ; le salicylate de bismuthyle C₆H₄CO₂(BiO)(OH).

La quantité de co-catalyseur utilisée, exprimée par la quantité de bismuth métallique contenue dans le co-catalyseur par rapport au poids du métal noble engagé, peut varier dans de larges limites. Par exemple, cette quantité peut être aussi petite que 0,1 % et peut atteindre le poids de métal noble engagé et même le dépasser sans inconvénient.

Plus particulièrement, cette quantité est choisie de manière qu'elle apporte dans le milieu d'oxydation de 10 à 900 ppm en poids de bismuth métallique par rapport à l'acide 2-hydroxybenzoïque hydroxyméthylé. A cet égard, des quantités supérieures de co-catalyseur de l'ordre de 900 à 1500 ppm peuvent naturellement être utilisées, mais sans avantage supplémentaire important.

Selon le procédé de l'invention, l'oxydation est conduite dans un milieu aqueux contenant en solution un agent alcalin. A cet égard, on fait appel en général, comme agent alcalin, à l'hydroxyde de sodium ou de potassium. La proportion de base minérale à utiliser est comprise entre 0,5 et 3 moles d'hydroxyde de sodium ou de potassium par rapport à l'acide 2-hydroxybenzoïque hydroxyméthylé.

La concentration de l'acide 2-hydroxybenzoïque hydroxyméthylé dans la solution aqueuse d'agent alcalin doit de préférence être telle que l'on évite toute précipitation et conserve une solution homogène.

La concentration pondérale de l'acide 2-hydroxybenzoïque hydroxyméthylé dans le milieu aqueux est habituellement comprise entre 1 % et 60 %, de préférence entre 2 % et 30 %.

Pratiquement une manière d'exécuter le procédé consiste à mettre en contact avec de l'oxygène moléculaire ou un gaz en contenant, par exemple l'air, la solution aqueuse renfermant l'acide 2-hydroxybenzoïque hydroxyméthylé à oxyder, l'agent alcalin, le catalyseur à base de platine ou de palladium, éventuellement le co-catalyseur à base de dérivé du bismuth, selon les proportions indiquées ci-dessus.

On opère à pression atmosphérique, mais on peut aussi le cas échéant opérer sous pression entre 1 et 20 bars.

Le mélange est ensuite agité à la température désirée jusqu'à consommation d'une quantité d'oxygène correspondant à celle nécessaire pour transformer la fonction alcool en aldéhyde. On contrôle donc la marche de la réaction, par la mesure de la quantité d'oxygène absorbée.

La température réactionnelle à adopter varie selon la stabilité thermique des produits à préparer.

D'une façon générale, la réaction est conduite dans une gamme de température allant de 50°C à 100°C, de préférence allant de 60°C à 80°C.

En fin de réaction qui dure de préférence, entre 30 minutes et 2 heures, on récupère l'acide 2-hydroxybenzoïque formylé en position 5 et substitué, au moins en position 3 par un groupe alkoxy et répondant préférentiellement à la formule (IV).

Puis, après refroidissement s'il y a lieu, on sépare la masse catalytique de la masse réactionnelle, par exemple par filtration.

Dans la dernière étape du procédé de l'invention, on effectue une réaction de décarboxylation.

Pour ce faire, on acidifie le liquide résultant par addition d'un acide protonique d'origine minérale, de préférence l'acide chlorhydrique ou l'acide sulfurique jusqu'à obtention d'un pH inférieur ou égal à 3, de préférence, entre 0 et 3.

On chauffe le milieu réactionnel à une température variant par exemple entre 120°C et 350°C et, de préférence, de 150°C et 220°C.

Le procédé est conduit, de préférence, sous la pression autogène des réactifs.

En fin de réaction, on refroidit le milieu réactionnel entre 20°C et 80°C.

On obtient un milieu bi-phasique constitué d'une phase organique comprenant d'une part, le 4-hydroxybenzaldéhyde substitué, au moins en position 3 par un groupe alkoxy, et répondant préférentiellement à la formule (V) et éventuellement le substrat de départ de formule (I) et d'autre part une phase aqueuse saline.

On sépare les phases organique et aqueuse et l'on récupère le 4-hydroxybenzaldéhyde sustitué à partir de la phase organique, selon les techniques classiques de séparation, de préférence, par distillation.

Comme mentionné précédemment, le procédé de l'invention est particulièrement bien adapté pour la préparation de la vanilline et de l'éthylvanilline.

On donne ci-après des exemples de réalisation de l'invention. Ces exemples sont donnés à titre illustratif et sans caractère limitatif.

### EXEMPLES

### Exemple 1

### 1 - Carboxylation du gaïacolate de potassium sous pression de CO₂.

On charge dans un réacteur Burton Corbelin de 500 mi en hastelloy B₂, équipé d'une turbine à pales, 224 g (1,81 mol) de gaïacol.

On ajoute 31,5 g (228 mmol) de carbonate de potassium.

On purge le réacteur par un courant de CO₂. On note une légère exothermie.

On chauffe à 170°C pendant 7 heures, tout en maintenant la pression de CO₂ à 20 bars.

Après refroidissement du réacteur à température ambiante, on ajoute 200 ml d'eau.

On coule une solution d'acide chlorhydrique 5 N jusqu'à obtention d'un pH d'environ 7,0. Il y a démixtion.

On décante la phase organique constituée essentiellement de gaïacol.

La phase aqueuse est acidifée à pH 1 avec de l'acide chlorhydrique. Il y a précipitation d'acide orthovanillique.

On sépare par filtration. On lave à l'eau et l'on sèche le produit obtenu à 40°C, sous pression réduite de 20 mm de mercure.

On récupère 38 g d'acide orthovanillique titrant 96 % en poids.

Le rendement est de 96 % par rapport au carbonate de potassium.

### 2 - Condensation acide orthovanillique/paraformaldéhyde.

A une suspension de 16,8 g (0,1 mol) d'acide orthovanillique dans 16,72 g d'eau, on ajoute 14,66 g d'une solution aqueuse d'hydroxyde de sodium à 30 % (0,11 mol) sous agitation et en chauffant.

Quand le milieu est homogène et à la température de 70°C, on ajoute 3 g de paraformaldéhyde (0,1 mol).

Après 6 heures d'agitation à 70°C, on dose la solution violette obtenue par chromatographie liquide haute performance :

Les résultats obtenus sont les suivants :
- TT (acide o-vanillique) = nb de moles d'acide o-vanillique transformées/nb de moles d'acide o-vanillique engagées = 42,5 %
- RR (acide 3-méthoxy-5-hydroxyméthylsalicylique) = nb de moles d'acide 3-méthoxy-5-hydroxyméthylsalicylique formées/ nb de moles d'acide o-vanillique engagées = 29,95 %
- RT = nb de moles d'acide 3-méthoxy-5-hydroxyméthylsalicylique formées/ nb de moles d'acide o-vanillique transformées = 70,5 %.

### 3 - Oxydation.

A la solution précédente, on ajoute 1,63 g de platine sur charbon à 2,5 % (0,2 mol %), puis 140 mg de sulfate de bismuth (0,2 mol %).

La température est ajustée à 65 °C et le pH à 12. Ce dernier sera maintenu à cette valeur au cours de la réaction par l'ajout de soude 30 %.

On envoie de l'oxygène dans le réacteur sous forte agitation à un débit de 1,5 l/h.

Après 3 heures, on ajoute 0,815 g de platine sur charbon, et l'on poursuit la réaction pendant 3 heures supplémentaires.

A ce moment, le dosage de la masse réactionnelle par chromatographie liquide haute performance donne :
- TT (acide 3-méthoxy-5-hydroxyméthylsalicylique) = nb de moles d'acide 3-méthoxy-5-hydroxyméthylsalicylique transformées/nb de moles d'acide 3-méthoxy-5-hydroxyméthylsalicylique engagées = 100 %
- RR (5-carboxyvanilline)= nb de moles de 5-carboxyvanilline formées/ nb de moles d'acide 3-méthoxy-5-hydroxyméthylsalicylique engagées = 59 %.

### 4 - Décarboxylation.

On dilue le milieu réactionnel issu de l'oxydation par 100 ml d'eau puis on le charge dans un réacteur Burton Corbelin en hastelloy B₂.

On coule une solution d'acide sulfurique 2 N jusqu'à obtention d'un pH d'environ 1,9.

On purge le réacteur sous un courant d'azote puis on chauffe pendant 30 mn à 200°C.

On refroidit rapidement par un courant d'eau froide.

On dilue le mélange réactionnel avec de l'acétonitrile, puis on le dose par chromatographie liquide haute performance.

Les résultats obtenus sont les suivants :
- TT 5-carboxyvanilline = nb de moles de 5-carboxyvanilline transformées/nb de moles de 5-carboxyvanilline engagées = 100 %
- RT vanilline = nb de moles de vanilline formées/nb de moles de 5-carboxyvanilline transformées = 99,4 %
- RR vanilline/acide o.vanillique = nb de moles de vanilline formées/nb de moles d'acide o-vanillique engagées = 27,4 %.

### Exemples 2 à 4

On effectue une série d'essais de décarboxylation du 3-carboxy-4-hydroxy 5-méthoxybenzaldéhyde.

On charge dans un réacteur Burton Corbelin de 50 ml en hastelloy B₂, équipé d'une turbine à pâle : 0,246 g (1,26 mmol) de 5-carboxyvanilline et 20 ml d'un mélange d'acide acétique et d'eau (50/50 en volume) dans l'exemple 2, 20 ml d'eau dans l'exemple 3 et 20 ml d'une solution d'acide sulfurique (5 mmol/l) dans l'exemple 4.

On purge le réacteur sous un courant d'azote.

On chauffe pendant 20 mn, à 160°C dans les exemples 2 et 3 et 200°C dans l'exemple 4.

On dose par chromatographie liquide haute performance le milieu réactionnel après dilution par l'acétonitrile : colonne Lichro Cart RP 18 - 5 µm - 250/4 mm commercialisée par Merck - éluant : 800 ml H₂O/200 ml CH₃CN/3,5 ml H₃PO₄- débit : 1 ml.mn⁻¹ - détection UV : 240 µm - température ambiante.

Les résultats sont consignés dans le tableau (I) suivant :

**Tableau (I)**

| Ref. exemple | Nature du solvant | Température (°C) | Durée (h) | TT 5-carboxy-vanilline (%) | RT vanilline (%) |
|---|---|---|---|---|---|
| 2 | CH₃COOH/H₂O | 160 | 7 h 05 | 89,6 | 95,1 |
| 3 | H₂O | 160 | 6 h 35 | 96,5 | 91,1 |
| 4 | H₂SO₄ | 200 | 0 h 20 | 98,9 | 98,4 |

## Revendications

1. Procédé de préparation d'un 4-hydroxybenzaldéhyde substitué, au moins en position 3 par un groupe alkoxy, caractérisé par le fait qu'il consiste à soumettre un composé phénolique substitué au moins en position 2 par un groupe alkoxy et dont les positions 4 et 6 sont libres, à une première étape de carboxylation en position 6 ; puis à une étape d'hydroxyméthylation en position 4, suivie d'une étape d'oxydation du groupe hydroxyméthyle en groupe formyle ; enfin à une dernière étape de décarboxylation.

2. Procédé selon la revendication 1 caractérisé par le fait que le composé phénolique substitué répond à la formule générale (I): 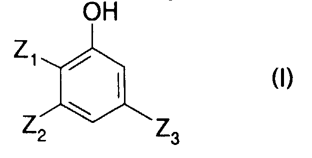 dans ladite formule (I) :
- Z₁ représente :
. un radical alkoxy linéaire ou ramifié ayant de 1 à 12 atomes de carbone, de préférence de 1 à 4 atomes de carbone tel que les radicaux méthoxy, éthoxy, propoxy, isopropoxy, butoxy, isobutoxy, sec-butoxy, tert-butoxy,
- Z₂ et Z₃, identiques ou différents, représentent un atome d'hydrogène ou l'un des groupes suivants :
. un radical alkyle, linéaire ou ramifié, ayant de 1 à 12 atomes de carbone, de préférence de 1 à 4 atomes de carbone, tel que méthyle, éthyle, propyle, isopropyle, butyle, isobutyle, sec-butyle, tert-butyle,
. un radical alcényle, linéaire ou ramifié, ayant de 2 à 12 atomes de carbone, de préférence de 2 à 4 atomes de carbone, tel que vinyle, allyle,
. un radical alkoxy linéaire ou ramifié ayant de 1 à 12 atomes de carbone, de préférence de 1 à 4 atomes de carbone tel que les radicaux méthoxy, éthoxy, propoxy, isopropoxy, butoxy, isobutoxy, sec-butoxy, tert-butoxy,
. un radical phényle,
. un atome d'halogène, de préférence un atome de fluor, chlore ou brome.

3. Procédé selon l'une des revendications 1 et 2 caractérisé par le fait que le composé phénolique substitué répond à la formule (I) dans laquelle Z₁ représente un radical alkoxy, linéaire ou ramifié, ayant de 1 à 6 atomes de carbone, de préférence, 1 à 4 atomes de carbone ; Z₂ et Z₃ représentant un atome d'hydrogène.

4. Procédé selon l'une des revendications 1 à 3 caractérisé par le fait que le composé phénolique substitué est le gaïacol ou le guétol.

5. Procédé selon l'une des revendications 1 à 4 caractérisé par le fait que l'on effectue dans une première étape, la carboxylation d'un composé phénolique substitué de formule (I), par réaction dudit composé sous forme salifiée avec du dioxyde de carbone.

6. Procédé selon la revendication 5 caractérisé par le fait que le composé phénolique substitué est sous forme salifiée, de préférence, sous forme des sels des éléments métalliques du groupe (la) de la classification périodique, de préférence, sous forme de sels de sodium ou de potassium.

7. Procédé selon l'une des revendications 5 et 6 caractérisé par le fait que le composé phénolique substitué sous forme salifiée est préparé en faisant réagir ledit composé avec une base, de préférence, l'hydroxyde de sodium ou de potassium ou un hydroxyde d'ammonium quaternaire puis en éliminant l'eau formée au cours de la réaction de salification ou par ajout d'un carbonate alcalin anhydre, de préférence, le carbonate de potassium.

8. Procédé selon l'une des revendications 5 à 7 caractérisé par le fait que la température à laquelle a lieu la carboxylation est comprise entre 130°C et 250°C.

9. Procédé selon l'une des revendications 5 à 8 caractérisé par le fait que la pression de dioxyde de carbone varie entre la pression atmosphérique et environ 100 bar, de préférence, entre 1 et 20.

10. Procédé selon l'une des revendications 5 à 9 caractérisé par le fait que la première étape du procédé de l'invention consiste à mettre en oeuvre le composé phénolique substitué, la base, à éliminer l'eau si nécessaire par distillation, puis à introduire le dioxyde de carbone.

11. Procédé de préparation d'un acide 2-hydroxybenzoïque hydroxyméthylé en position 5 substitué, au moins en position 3 par un groupe alkoxy caractérisé par le fait qu'il consiste à faire réagir un acide 2-hydroxybenzoïque substitué, au moins en position 3 par un groupe alkoxy, avec du formaldéhyde ou un générateur de formaldéhyde, éventuellement en présence d'une base.

12. Procédé selon la revendication 11 caractérisé par le fait que l'acide 2-hydroxybenzoïque substitué, au moins en position 3 par un groupe alkoxy, répond à la formule Générale (II) : 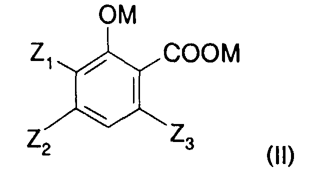 dans ladite formule (II) :
- Z₁, Z₂, Z₃, ayant les significations données dans les revendications 2 et 3,
- M représente un atome d'hydrogène et/ou un cation métallique du groupe (Ia).

13. Procédé selon l'une des revendications 11 et 12 caractérisé par le fait que le générateur de formaldéhyde est le trioxane ou le paraformaldéhyde utilisé sous la forme de polyformaldéhydes linéaires de degré de polymérisation indifférent, ayant de préférence un nombre de motifs (CH₂O) compris entre 8 et 100 motifs.

14. Procédé selon l'une des revendications 11 à 13 caractérisé par le fait que le rapport molaire formaldéhyde/sel d'acide hydroxybenzoïque se situe entre 0,5 et 3,0.

15. Procédé selon l'une des revendications 11 à 14 caractérisé par le fait que la température de la réaction varie de 50°C à 100°C et, de préférence, de 60°C à 80°C.

16. Procédé selon l'une des revendications 11 à 15 caractérisé par le fait que l'acide 2-hydroxybenzoïque hydroxyméthylé en position 5 et substitué, au moins en position 3 par un groupe alkoxy répond à la formule générale (III) : 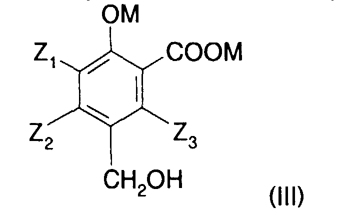 dans ladite formule (III) :
- Z₁, Z₂ et Z₃ ont les significations données dans les revendications 2 et 3,
- M représente un atome d'hydrogène et/ou un cation métallique du groupe (la).

17. Procédé selon la revendication 16 caractérisé par le fait que l'acide 2-hydroxybenzoïque hydroxyméthylé en position 5 et substitué, au moins en position 3 par un groupe alkoxy, répond à la formule générale (III) dans laquelle Z₁ représente un radical alkoxy, linéaire ou ramifié, ayant de 1 à 6 atomes de carbone, de préférence, 1 à 4 atomes de carbone, et encore plus préférentiellement un radical méthoxy ou éthoxy ; Z₂ et Z₃ représentant un atome d'hydrogène.

18. Procédé de préparation d'un acide 2-hydroxybenzoïque formylé en position 5 et substitué, au moins en position 3 par un groupe alkoxy, caractérisé par le fait qu'il consiste à oxyder l'acide 2-hydroxybenzoïque hydroxyméthylé en position 5 et substitué, au moins en position 3 par un groupe alkoxy, en phase liquide, à l'aide d'oxygène moléculaire ou un gaz en contenant, en opérant en milieu aqueux renfermant un agent alcalin, en présence d'un catalyseur à base de platine ou de palladium éventuellement en présence d'un co-catalyseur à base d'un dérivé du bismuth.

19. Procédé selon la revendication 18 caractérisé par le fait que l'acide 2-hydroxybenzoïque hydroxyméthylé en position 5 et substitué, au moins en position 3 par un groupe alkoxy, répond à la formule générale (III) décrit dans l'une des revendications 16 et 17.

20. Procédé selon l'une des revendications 18 et 19 caractérisé par le fait que le catalyseur est choisi parmi : le noir de platine, le noir de palladium, l'oxyde de platine, l'oxyde de palladium ou le métal noble lui-même déposé sur des supports divers tels que le noir de carbone, le carbonate de calcium, les alumines et silices activées ou des matériaux équivalents.

21. Procédé selon l'une des revendications 18 à 20 caractérisé par le fait que la quantité de ce catalyseur à mettre en oeuvre, exprimée en poids de platine ou de palladium par rapport à celui de l'acide 2-hydroxybenzoïque hydroxyméthylé, peut varier de 0,01 à 4 % et, de préférence, de 0,04 à 2 %.

22. Procédé selon l'une des revendications 18 à 21 caractérisé par le fait que l'on fait appel, comme co-catalyseur, à un dérivé minéral ou organique du bismuth dans lequel l'atome de bismuth se trouve à un degré d'oxydation supérieur à zéro, par exemple égal à 2, 3, 4 ou 5.

23. Procédé selon la revendication 22 caractérisé par le fait que le dérivé du bismuth est pris dans le groupe formé par : les oxydes de bismuth ; les hydroxydes de bismuth ; les sels de bismuth ou de bismuthyle d'hydracides minéraux, de préférence les chlorure, bromure, iodure, sulfure, sélénure, tellure ; les sels de bismuth ou de bismuthyle d'oxyacides minéraux, de préférence les sulfite, sulfate, nitrite, nitrate, phosphite, phosphate, pyrophosphate, carbonate, perchlorate, antimoniate, arséniate, sélénite, séléniate ; les sels de bismuth ou de bismuthyle d'acides organiques aliphatiques ou aromatiques, de préférence les acétate, propionate, salicylate, benzoate, oxalate, tartrate, lactate, citrate ; les phénates de bismuth ou de bismuthyle, de préférence les gallate et pyrogallate.

24. Procédé selon la revendication 23 caractérisé par le fait que le dérivé du bismuth est pris dans le groupe formé par : les oxydes de bismuth Bi₂O₃ et Bi₂O₄ ; l'hydroxyde de bismuth Bi(OH)₃ ; le chlorure de bismuth BiCl₃ ; le bromure de bismuth BiBr₃ ; l'iodure de bismuth Bil₃ ; le sulfate neutre de bismuth Bi₂(SO₄)₃ ; le nitrate neutre de bismuth Bi(NO₃)₃, 5H₂O ; le nitrate de bismuthyle BiO(NO₃) ; le carbonate de bismuthyle (BiO)₂CO₃, 0,5 H₂O ; l'acétate de bismuth Bi(C₂H₃O₂)₃ ; le salicylate de bismuthyle C₆H₄CO₂(BiO)OH.

25. Procédé selon l'une des revendications 22 à 24 caractérisé par le fait que la quantité de co-catalyseur utilisée est choisie de manière qu'elle apporte dans la milieu : d'une part, au moins 0,1 % en poids de bismuth métallique par rapport au poids du métal noble engagé, et d'autre part de 10 à 800 ppm en poids de bismuth métallique par rapport à l'acide 2-hydroxybenzoïque hydroxyméthylé.

26. Procédé selon l'une des revendications 18 à 25 caractérisé par le fait que la réaction d'oxydation est opérée au sein d'un milieu aqueux renfermant de 0,5 à 3 moles d'hydroxyde de sodium ou de potassium par rapport à l'acide 2-hydroxybenzoïque hydroxyméthylé.

27. Procédé selon l'une des revendications 18 à 26 caractérisé par le fait que la réaction d'oxydation est conduite à une température allant de 50°C à 100°C, de préférence allant de 60°C à 80°C.

28. Procédé selon l'une des revendications 18 à 27 caractérisé par le fait que l'on refroidit le milieu réactionnel et que l'on sépare le catalyseur.

29. Procédé selon l'une des revendications 18 à 28 caractérisé par le fait que l'on effectue la décarboxylation l'acide 2-hydroxybenzoïque formylé en position 5 et substitué, au moins en position 3, par un groupe alkoxy.

30. Procédé selon la revendication 29 caractérisé par le fait que l'acide 2-hydroxybenzoïque formylé en position 5 et substitué, au moins en position 3 par un Groupe alkoxy, répond à la formule générale (IV) : 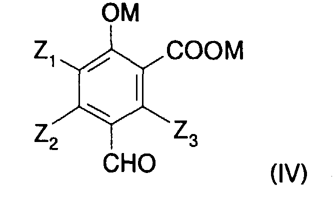 dans ladite formule (IV) :
- Z₁, Z₂, Z₃, et M ayant les significations données dans les revendications 2 et 3.

31. Procédé selon l'une des revendications 29 à 30 caractérisé par le fait que l'on effectue la décarboxylation dudit acide par addition d'un acide protonique d'origine minérale, de préférence l'acide chlorhydrique ou l'acide sulfurique jusqu'à obtention d'un pH inférieur ou égal à 3.

32. Procédé selon l'une des revendications 29 à 31 caractérisé par le fait que l'on chauffe le milieu réactionnel à une température variant entre 120°C et 350°C et, de préférence, de 150°C et 220°C et après refroidissement que l'on sépare le 4-hydroxybenzaldéhyde substitué, au moins en position 3 par un groupe alkoxy, et répondant préférentiellement à la formule (V): 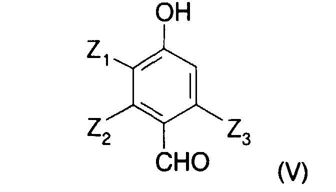 dans ladite formule (V):
- Z₁, Z₂, Z₃, et M ayant les significations données dans les revendications 2 et 3.

## Claims

1. Process for the preparation of a substituted 4-hydroxybenzaldehyde, substituted at least in the 3 position by an alkoxy group, characterized in that it comprises subjecting a substituted phenol compound, substituted at least in the 2 position by an alkoxy group and in which the 4 and 6 positions are free, to a first stage of carboxylation in the 6 position, then to a stage of hydroxymethylation in the 4 position, followed by a stage of oxidation of the hydroxymethyl group to a formyl group, and finally to a last decarboxylation stage.

2. Process according to Claim 1, characterized in that the substituted phenol compound corresponds to the general formula (I): 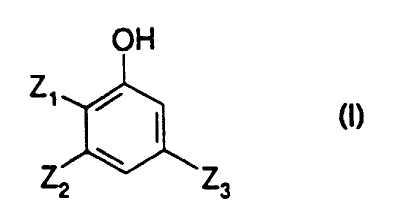 in the said formula (I):
- Z₁ represents:
. a linear or branched alkoxy radical having from 1 to 12 carbon atoms, preferably from 1 to 4 carbon atoms, such as the methoxy, ethoxy, propoxy, isopropoxy, butoxy, isobutoxy, sec-butoxy and tert-butoxy radicals,
- Z₂ and Z₃, which are identical or different, represent a hydrogen atom or one of the following groups:
. a linear or branched alkyl radical having from 1 to 12 carbon atoms, preferably from 1 to 4 carbon atoms, such as methyl, ethyl, propyl, isopropyl, butyl, isobutyl, sec-butyl or tert-butyl,
. a linear or branched alkenyl radical having from 2 to 12 carbon atoms, preferably from 2 to 4 carbon atoms, such as vinyl or allyl,
. a linear or branched alkoxy radical having from 1 to 12 carbon atoms, preferably from 1 to 4 carbon atoms, such as the methoxy, ethoxy, propoxy, isopropoxy, butoxy, isobutoxy, sec-butoxy and tert-butoxy radicals,
. a phenyl radical,
. a halogen atom, preferably a fluorine, chlorine or bromine atom.

3. Process according to either of Claims 1 and 2, characterized in that the substituted phenol compound corresponds to the formula (I) in which Z₁ represents a linear or branched alkoxy radical having from 1 to 6 carbon atoms, preferably 1 to 4 carbon atoms, Z₂ and Z₃ representing a hydrogen atom.

4. Process according to one of Claims 1 to 3, characterized in that the substituted phenol compound is guaiacol or 2-ethoxyphenol.

5. Process according to one of Claims 1 to 4, characterized in that, in a first stage, a substituted phenol compound of formula (I) is carboxylated by reaction of the said compound in the salified form with carbon dioxide.

6. Process according to Claim 5, characterized in that the substituted phenol compound is in the salified form, preferably in the form of salts of metal elements from group (Ia) of the periodic classification, preferably in the form of sodium or potassium salts.

7. Process according to either of Claims 5 and 6, characterized in that the substituted phenol compound in the salified form is prepared by reacting the said compound with a base, preferably sodium or potassium hydroxide, or a quaternary ammonium hydroxide, and by then removing the water formed during the salification reaction or by addition of an anhydrous alkaline carbonate, preferably potassium carbonate.

8. Process according to one of Claims 5 to 7, characterized in that the temperature at which the carboxylation takes place is between 130°C and 250°C.

9. Process according to one of Claims 5 to 8, characterized in that the carbon dioxide pressure varies between atmospheric pressure and approximately 100 bar, preferably between 1 and 20.

10. Process according to one of Claims 5 to 9, characterized in that the first stage of the process of the invention comprises the use of the substituted phenol compound and the base, the removal of the water, if necessary, by distillation, and then the introduction of the carbon dioxide.

11. Process for the preparation of a 2-hydroxybenzoic acid which is hydroxymethylated in the 5 position and which is substituted at least in the 3 position by an alkoxy group, characterized in that it comprises the reaction of a substituted 2-hydroxybenzoic acid, substituted at least in the 3 position by an alkoxy group, with formaldehyde or a formaldehyde generator, optionally in the presence of a base.

12. Process according to Claim 11, characterized in that the substituted 2-hydroxybenzoic acid, substituted at least in the 3 position by an alkoxy group, corresponds to the general formula (II): 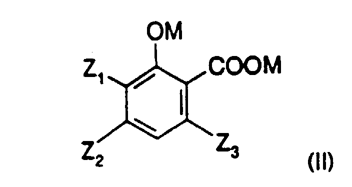 in the said formula (II):
- Z₁, Z₂ and Z₃ having the meanings given in Claims 2 and 3,
- M represents a hydrogen atom and/or a metal cation from Group (Ia).

13. Process according to either of Claims 11 and 12, characterized in that the formaldehyde generator is trioxane or paraformaldehyde used in the form of linear polyformaldehydes in which the degree of polymerization is immaterial, preferably having a number of (CH₂O) units of between 8 and 100 units.

14. Process according to one of Claims 11 to 13, characterized in that the formaldehyde/ hydroxybenzoic acid salt molar ratio lies between 0.5 and 3.0.

15. Process according to one of Claims 11 to 14, characterized in that the temperature of the reaction varies from 50°C to 100°C and preferably from 60°C to 80°C.

16. Process according to one of Claims 11 to 15, characterized in that the 2-hydroxybenzoic acid which is hydroxymethylated in the 5 position and which is substituted at least in the 3 position by an alkoxy group corresponds to the general formula (III) : 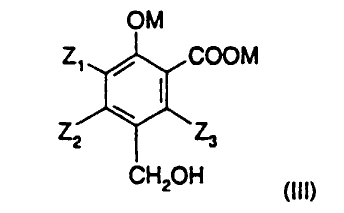 in the said formula (III):
- Z₁, Z₂ and Z₃ have the meanings given in Claims 2 and 3,
- M represents a hydrogen atom and/or a metal cation from Group (Ia).

17. Process according to Claim 16, characterized in that the 2-hydroxybenzoic acid which is hydroxymethylated in the 5 position and which is substituted at least in the 3 position by an alkoxy group corresponds to the general formula (III) in which Z₁ represents a linear or branched alkoxy radical having from 1 to 6 carbon atoms, preferably 1 to 4 carbon atoms, and more preferentially still a methoxy or ethoxy radical, Z₂ and Z₃ representing a hydrogen atom.

18. Process for the preparation of a 2-hydroxybenzoic acid which is formylated in the 5 position and which is substituted at least in the 3 position by an alkoxy group, characterized in that it comprises the oxidation of 2-hydroxybenzoic acid which is hydroxymethylated in the 5 position and which is substituted at least in the 3 position by an alkoxy group, in the liquid phase, using molecular oxygen or a gas containing it, the oxidation being carried out in aqueous medium containing an alkaline agent, in the presence of a catalyst based on platinum or on palladium, optionally in the presence of a cocatalyst based on a bismuth derivative.

19. Process according to Claim 18, characterized in that the 2-hydroxybenzoic acid which is hydroxymethylated in the 5 position and which is substituted at least in the 3 position by an alkoxy group corresponds to the general formula (III) described in either of Claims 16 and 17.

20. Process according to either of Claims 18 and 19, characterized in that the catalyst is chosen from: platinum black, palladium black, platinum oxide, palladium oxide or the nobel metal itself deposited on various supports such as carbon black, calcium carbonate, activated aluminas and silicas or equivalent materials.

21. Process according to one of Claims 18 to 20, characterized in that the amount of this catalyst to be used, expressed as weight of platinum or palladium with respect to that of the hydroxymethylated 2-hydroxybenzoic acid, can vary from 0.01 to 4 % and preferably from 0.04 to 2 %.

22. Process according to one of Claims 18 to 21, characterized in that use is made, as cocatalyst, of an inorganic or organic bismuth derivative in which the bismuth atom has an oxidation number greater than zero, for example equal to 2, 3, 4 or 5.

23. Process according to Claim 22, characterized in that the bismuth derivative is taken from the group formed by: bismuth oxides, bismuth hydroxides, bismuth or bismuthyl salts of inorganic hydracids, preferably the chloride, bromide, iodide, sulphide, selenide and telluride, bismuth or bismuthyl salts of inorganic oxyacids, preferably the sulphite, sulphate, nitrite, nitrate, phosphite, phosphate, pyrophosphate, carbonate, perchlorate, antimonate, arsenate, selenite and selenate, bismuth or bismuthyl salts of aliphatic or aromatic organic acids, preferably the acetate, propionate, salicylate, benzoate, oxalate, tartrate, lactate and citrate, or bismuth or bismuthyl phenoxides, preferably the gallate and pyrogallate.

24. Process according to Claim 23, characterized in that the bismuth derivative is taken from the group formed by: bismuth oxides Bi₂O₃ and Bi₂O₄, bismuth hydroxide Bi(OH)₃, bismuth chloride BiCl₃, bismuth bromide BiBr₃, bismuth iodide BiI₃, neutral bismuth sulphate Bi₂(SO₄)₃, neutral bismuth nitrate Bi(NO₃)₃·5H₂O, bismuthyl nitrate BiO(NO₃), bismuthyl carbonate (BiO)₂CO₃·0.5H₂O, bismuth acetate Bi(C₂H₃O₂)₃ or bismuthyl salicylate C₆H₄CO₂(BiO)OH.

25. Process according to one of Claims 22 to 24, characterized in that the amount of cocatalyst used is chosen so that it introduces into the mixture: on the one hand, at least 0.1 % by weight of metallic bismuth with respect to the weight of the noble metal used and, on the other hand, from 10 to 800 ppm by weight of metallic bismuth with respect to the hydroxymethylated 2-hydroxybenzoic acid.

26. Process according to one of Claims 18 to 25, characterized in that the oxidation reaction is carried out in an aqueous medium containing from 0.5 to 3 mol of sodium or potassium hydroxide with respect to the hydroxymethylated 2-hydroxybenzoic acid.

27. Process according to one of Claims 18 to 26, characterized in that the oxidation reaction is carried out at a temperature ranging from 50°C to 100°C, preferably ranging from 60°C to 80°C.

28. Process according to one of Claims 18 to 27, characterized in that the reaction mixture is cooled and in that the catalyst is separated.

29. Process according to one of Claims 18 to 28, characterized in that the 2-hydroxybenzoic acid which is formylated in the 5 position and which is substituted at least in the 3 position by an alkoxy group is decarboxylated.

30. Process according to Claim 29, characterized in that the 2-hydroxybenzoic acid which is formylated in the 5 position and which is substituted at least in the 3 position by an alkoxy group corresponds to the general formula (IV) : 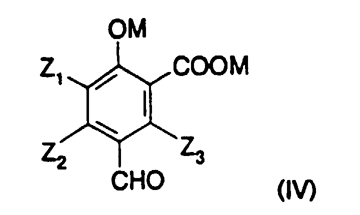 in the said formula (IV):
- Z₁, Z₂, Z₃ and M having the meanings given in Claims 2 and 3.

31. Process according to either of Claims 29 and 30, characterized in that the said acid is decarboxylated by addition of a proton acid of inorganic origin, preferably hydrochloric acid or sulphuric acid, until a pH less than or equal to 3 is obtained.

32. Process according to one of Claims 29 to 31, characterized in that the reaction mixture is heated at a temperature varying between 120°C and 350°C and preferably between 150°C and 220°C and, after cooling, in that the substituted 4-hydroxybenzaldehyde, substituted at least in the 3 position by an alkoxy group, preferentially corresponding to the formula (V): 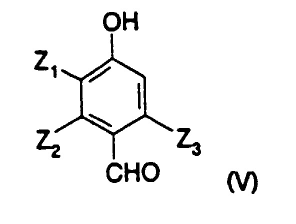 in the said formula (V):
- Z₁, Z₂, Z₃ and M having the meanings given in Claims 2 and 3,
is separated.

## Patentansprüche

1. Verfahren zur Herstellung eines 4-Hydroxybenzaldehyds, der mindestens in 3-Stellung mit einer Alkoxygruppe substituiert ist, dadurch gekennzeichnet, daß es darin besteht, eine Phenolverbindung, die mindestens in 2-Stellung mit einer Alkoxygruppe substituiert ist und deren 4- und 6-Stellung frei sind, in einem ersten Schritt einer Carboxylierung in 6-Stellung zu unterziehen; dann einem Schritt der Hydroxymethylierung in 4-Stellung, gefolgt von einem Schritt der Oxidation der Hydroxymethylgruppe zur Formylgruppe; schließlich einem letzten Schritt der Decarboxylierung.

2. Verfahren nach Anspruch 1, dadurch gekennzeichnet, daß die substituierte Phenolverbindung der allgemeinen Formel (I) entspricht: 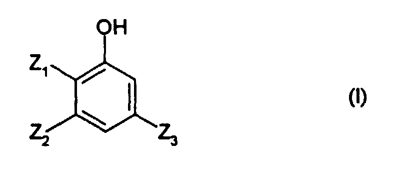 wobei in der genannten Formel (I):
- Z₁ einen linearen oder verzweigten Alkoxyrest mit 1 bis 12 Kohlenstoffatomen, vorzugsweise 1 bis 4 Kohlenstoffatomen, bedeutet, wie z.B. die Methoxy-, Ethoxy-, Propoxy-, Isopropoxy-, Butoxy-, Isobutoxy-, sec.-Butoxy-, tert.-Butoxy-Reste,
- Z₂ und Z₃ gleich oder verschieden ein Wasserstoffatom oder eine der folgenden Gruppen bedeuten:
• einen linearen oder verzweigten Alkylrest mit 1 bis 12 Kohlenstoffatomen, vorzugsweise 1 bis 4 Kohlenstoffatomen, wie z.B. Methyl, Ethyl, Propyl, Isopropyl, Butyl, Isobutyl, sec.-Butyl, tert.-Butyl,
• einen linearen oder verzweigten Alkenylrest mit 2 bis 12 Kohlenstoffatomen, vorzugsweise 2 bis 4 Kohlenstoffatomen, wie z.B. Vinyl, Allyl,
• einen linearen oder verzweigten Alkoxyrest mit 1 bis 12 Kohlenstoffatomen, vorzugsweise 1 bis 4 Kohlenstoffatomen, wie z.B. die MethoxyEthoxy-, Propoxy-, Isopropoxy-, Butoxy-, Isobutoxy-, sec.-Butoxy-, tert.-Butoxy-Reste,
• einen Phenylrest,
• ein Halogenatom, vorzugsweise ein Fluor-, Chlor- oder Bromatom.

3. Verfahren nach einem der Ansprüche 1 und 2, dadurch gekennzeichnet, daß die substituierte Phenolverbindung der Formel (I) entspricht, in der Z₁ ein linearer oder verzweigter Alkoxyrest mit 1 bis 6 Kohlenstoffatomen, vorzugsweise 1 bis 4 Kohlenstoffatomen ist; Z₂ und Z₃ ein Wasserstoffatom bedeuten.

4. Verfahren nach einem der Ansprüche 1 bis 3, dadurch gekennzeichnet, daß die substituierte Phenolverbindung Guajakol oder 2-Ethoxyphenol ist.

5. Verfahren nach einem der Ansprüche 1 bis 4, dadurch gekennzeichnet, daß man in einem ersten Schritt die Carboxylierung einer substituierten Phenolverbindung der Formel (I) durch Reaktion der genannten Verbindung in der Salzform mit Kohlendioxid durchführt.

6. Verfahren nach Anspruch 5, dadurch gekennzeichnet, daß die substituierte Phenolverbindung in Salzform vorliegt, vorzugsweise in Form von Salzen der metallischen Elemente der Gruppe (Ia) des Periodensystems, vorzugsweise in Form von Natrium- oder Kaliumsalzen.

7. Verfahren nach einem der Ansprüche 5 und 6, dadurch gekennzeichnet, daß die in Salzform vorliegende substituierte Phenolverbindung hergestellt wird, indem man die genannte Verbindung mit einer Base, vorzugsweise Natrium- oder Kaliumhydroxid oder ein quartäres Ammoniumhydroxid, reagieren läßt und anschließend das während der Salzbildungsreaktion oder der Zugabe eines wasserfreien Alkalicarbonats, vorzugsweise Kaliumcarbonat, gebildete Wasser entfernt.

8. Verfahren nach einem der Ansprüche 5 bis 7, dadurch gekennzeichnet, daß die Temperatur, bei der die Carboxylierung stattfindet, zwischen 130 °C und 250 °C liegt.

9. Verfahren nach einem der Ansprüche 5 bis 8, dadurch gekennzeichnet, daß der Druck des Kohlendioxids zwischen atmosphärischem Druck und ungefähr 100 bar, vorzugsweise zwischen 1 und 20 bar, beträgt.

10. Verfahren nach einem der Ansprüche 5 bis 9, dadurch gekennzeichnet, daß der erste Schritt des erfindungsgemäßen Verfahrens darin besteht, die substituierte Phenolverbindung und die Base einzusetzen, das Wasser falls erforderlich durch Destillation zu entfernen und anschließend das Kohlendioxid zuzugeben.

11. Verfahren zur Herstellung einer in 5-Stellung hydroxymethylierten mindestens in 3-Stellung mit einer Alkoxygruppe substituierten 2-Hydroxybenzoesäure, dadurch gekennzeichnet, daß es darin besteht, die mindestens in 3-Stellung mit einer Alkoxygruppe substituierte 2-Hydroxybenzoesäure mit Formaldehyd oder einem Formaldehydbildner gegebenenfalls in Gegenwart einer Base reagieren zu lassen.

12. Verfahren nach Anspruch 11, dadurch gekennzeichnet, daß die substituierte 2-Hydroxybenzoesäure, die mindestens in 3-Stellung mit einer Alkoxygruppe substituiert ist, der allgemeinen Formel (II) entspricht: 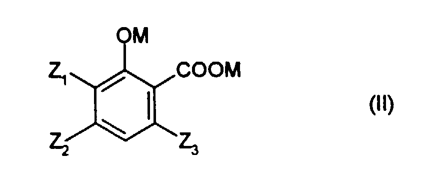 wobei in der genannten Formel (II):
- Z₁, Z₂ und Z₃ die in den Ansprüchen 2 und 3 gegebenen Bedeutungen haben,
- M ein Wasserstoffatom und/oder ein metallisches Kation der Gruppe (Ia) bedeutet.

13. Verfahren nach einem der Ansprüche 11 und 12, dadurch gekennzeichnet, daß der Formaldehydbildner Trioxan oder Paraformaldehyd ist, der in Form von linearen Polyformaldehyden eines unbestimmten Polymerisationsgrads verwendet wird und vorzugsweise eine Anzahl von (CH₂O)-Einheiten zwischen 8 und 100 Einheiten hat.

14. Verfahren nach einem der Ansprüche 11 bis 13, dadurch gekennzeichnet, daß das Molverhältnis Formaldehyd/Salz der Hydroxybenzoesäure zwischen 0,5 und 3,0 liegt.

15. Verfahren nach einem der Ansprüche 11 bis 14, dadurch gekennzeichnet, daß die Reaktionstemperatur von 50 °C bis 100 °C und vorzugsweise von 60 °C bis 80 °C reicht.

16. Verfahren nach einem der Ansprüche 11 bis 15, dadurch gekennzeichnet, daß die in 5-Stellung hydroxymethylierte, substituierte 2-Hydroxybenzoesäure, die mindestens in 3-Stellung mit einer Alkoxygruppe substituiert ist, der allgemeinen Formel (III) entspricht: 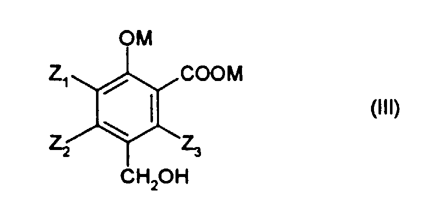 wobei in der genannten Formel (III) :
- Z₁, Z₂ und Z₃ die in den Ansprüchen 2 und 3 gegebenen Bedeutungen haben,
- M ein Wasserstoffatom und/oder ein metallisches Kation der Gruppe (Ia) bedeutet.

17. Verfahren nach Anspruch 16, dadurch gekennzeichnet, daß die in 5-Stellung hydroxymethylierte substituierte 2-Hydroxybenzoesäure, die mindestens in 3-Stellung mit einer Alkoxygruppe substituiert ist, der allgemeinen Formel (III) entspricht, in der Z₁ ein linearer oder verzweigter Alkoxyrest mit 1 bis 6 Kohlenstoffatomen, vorzugsweise 1 bis 4 Kohlenstoffatomen, und noch spezieller ein Methoxy- oder Ethoxyrest ist; Z₂ und Z₃ ein Wasserstoffatom bedeuten.

18. Verfahren zur Herstellung einer in 5-Stellung formylierten, substituierten 2-Hydroxybenzoesäure, die mindestens in 3-Stellung mit einer Alkoxygruppe substituiert ist, dadurch gekennzeichnet, daß es darin besteht, die in 5-Stellung hydroxymethylierte, substituierte 2-Hydroxybenzoesäure, die mindestens in 3-Stellung mit einer Alkoxygruppe substituiert ist, in flüssiger Phase mit Hilfe von molekularem Sauerstoff oder einem diesen enthaltenden Gas zu oxidieren, wobei in einem ein alkalisches Mittel enthaltenden wäßrigen Medium in Gegenwart eines Katalysators auf Platin- oder Palladiumbasis, gegebenenfalls in Gegenwart eines Cokatalysators auf Basis einer Bismutverbindung gearbeitet wird.

19. Verfahren nach Anspruch 18, dadurch gekennzeichnet, daß die in 5-Stellung hydroxymethylierte, substituierte 2-Hydroxybenzoesäure, die mindestens in 3-Stellung mit einer Alkoxygruppe substituiert ist, der in einem der Ansprüche 16 und 17 beschriebenen allgemeinen Formel (III) entspricht.

20. Verfahren nach einem der Ansprüche 18 und 19, dadurch gekennzeichnet, daß der Katalysator gewählt ist aus: Platinschwarz, Palladiumschwarz, Platinoxid, Palladiumoxid oder dem Edelmetall selbst, das auf verschiedenen Trägern wie Ruß, Calciumcarbonat, den Aluminiumoxiden und aktiven Kieselsäuren oder äquivalenten Materialien abgeschieden ist.

21. Verfahren nach einem der Ansprüche 18 bis 20, dadurch gekennzeichnet, daß die Menge des einzusetzenden Katalysators, definiert als das Gewicht von Platin oder Palladium im Verhältnis zu dem der hydroxymethylierten 2-Hydroxybenzoesäure, von 0,01 bis 4 % und vorzugsweise von 0,04 bis 2 % reicht.

22. Verfahren nach einem der Ansprüche 18 bis 21, dadurch gekennzeichnet, daß man als Cokatalysator eine mineralische oder organische Bismutverbindung verwendet, in der sich das Bismutatom in einem Oxidationsgrad größer 0, z.B. 2, 3, 4 oder 5, befindet.

23. Verfahren nach Anspruch 22, dadurch gekennzeichnet, daß die Bismutverbindung zur Gruppe gehört, die besteht aus: den Bismutoxiden; den Bismuthydroxiden; den Bismut- oder Bismutoxidsalzen der mineralischen Wasserstoffsäuren, vorzugsweise den Chloriden, Bromiden, Iodiden, Sulfiden, Seleniden, Telluriden; den Bismut- oder Bismutoxidsalzen der mineralischen Oxosäuren, vorzugsweise den Sulfiten, Sulfaten, Nitriten, Nitraten, Phosphiten, Phosphaten, Pyrophosphaten, Carbonaten, Perchloraten, Antimonaten, Arsenaten, Seleniten, Selenaten; den Bismut- oder Bismutoxidsalzen der aliphatischen oder aromatischen organischen Säuren, vorzugsweise den Acetaten, Propionaten, Salicylaten, Benzoaten, Oxalaten, Tartraten, Lactaten, Citraten; den Bismut- oder Bismutoxidphenolaten, vorzugsweise den Gallaten oder Pyrogallaten.

24. Verfahren nach Anspruch 23, dadurch gekennzeichnet, daß die Bismutverbindung zur Gruppe gehört, die besteht aus: den Bismutoxiden Bi₂O₃ und Bi₂O₄; Bismuthydroxid Bi(OH)₃; Bismutchlorid BiCl₃; Bismutbromid BiBr₃; Bismutiodid BiI₃; neutralem Bismutsulfat Bi₂(SO₄)₃; neutralem Bismutnitrat Bi(NO₃)₃ · 5H₂O; Bismutoxidnitrat BiO(NO₃); Bismutoxidcarbonat (BiO)₂CO₃ · 0,5H₂O; Bismutacetat Bi(C₂H₃O₂)₃; Bismutoxidsalicylat C₆H₄CO₂(BiO)OH.

25. Verfahren nach einem der Ansprüche 22 bis 24, dadurch gekennzeichnet, daß die Menge des verwendeten Cokatalysators derart gewählt ist, daß sie in das Medium einbringt: einerseits mindestens 0,1 Gew.-% metallisches Bismut im Verhältnis zum Gewicht des beteiligten Edelmetalls und andererseits 10 bis 800 Gew.-ppm metallisches Bismut im Verhältnis zur hydroxymethylierten 2-Hydroxybenzoesäure.

26. Verfahren nach einem der Ansprüche 18 bis 25, dadurch gekennzeichnet, daß die Oxidationsreaktion in einem wäßrigen Medium stattfindet, das 0,5 bis 3 mol Natrium- oder Kaliumhydroxid im Verhältnis zur hydroxymethylierten 2-Hydroxybenzoesäure enthält.

27. Verfahren nach einem der Ansprüche 18 bis 26, dadurch gekennzeichnet, daß die Oxidationsreaktion bei einer Temperatur von 50 °C bis 100 °C, vorzugsweise von 60 °C bis 80 °C, stattfindet.

28. Verfahren nach einem der Ansprüche 18 bis 27, dadurch gekennzeichnet, daß man das Reaktionsmilieu abkühlt und den Katalysator abtrennt.

29. Verfahren nach einem der Ansprüche 18 bis 28, dadurch gekennzeichnet, daß man die Decarboxylierung der in 5-Stellung formylierten substituierten 2-Hydroxybenzoesäure, die mindestens in 3-Stellung mit einer Alkoxygruppe substituiert ist, durchführt.

30. Verfahren nach Anspruch 29, dadurch gekennzeichnet, daß die in 5-Stellung formylierte substituierte 2-Hydroxybenzoesäure, die mindestens in 3-Stellung mit einer Alkoxygruppe substituiert ist, der allgemeinen Formel (IV) entspricht: 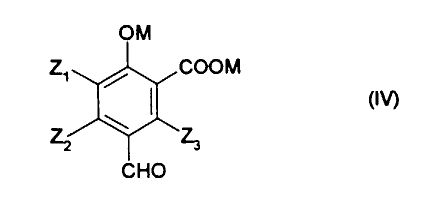 wobei in der genannten Formel (IV):
- Z₁, Z₂, Z₃ und M die in den Ansprüchen 2 und 3 genannten Bedeutungen haben.

31. Verfahren nach einem der Ansprüche 29 und 30, dadurch gekennzeichnet, daß man die Decarboxylierung der genannten Säure durch Addition einer Protonensäure mineralischen Ursprungs, vorzugsweise Salzsäure oder Schwefelsäure, bis zum Erreichen eines pH-Wertes kleiner oder gleich 3 durchführt.

32. Verfahren nach einem der Ansprüche 29 bis 31, dadurch gekennzeichnet, daß man das Reaktionsgemisch auf eine Temperatur zwischen 120 °C und 350 °C, vorzugsweise zwischen 150 °C und 220 °C, erwärmt und daß man nach dem Abkühlen das substituierte 4-Hydroxybenzaldehyd, das mindestens in 3-Stellung mit einer Alkoxygruppe substituiert ist, abtrennt, das vorzugsweise der Formel (V) entspricht: 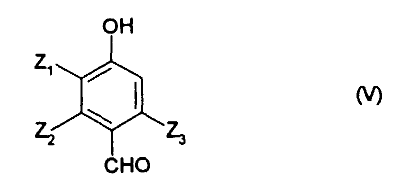 wobei in der genannten Formel (V):
- Z₁, Z₂, Z₃ und M die in den Ansprüchen 2 und 3 genannten Bedeutungen haben.
